**Europäisches Patentamt**

**European Patent Office**

**Office européen des brevets**

(11) Veröffentlichungsnummer: **0 134 777**
A2

(12) # EUROPÄISCHE PATENTANMELDUNG

(21) Anmeldenummer: **84890143.5**

(22) Anmeldetag: **30.07.84**

(51) Int. Cl.⁴: **A 61 L 2/24**
**A 61 L 2/06**

(30) Priorität: **04.08.83 AT 2823/83**

(43) Veröffentlichungstag der Anmeldung:
**20.03.85 Patentblatt 85/12**

(84) Benannte Vertragsstaaten:
**BE CH DE FR GB IT LI LU NL SE**

(71) Anmelder: **VEREINIGTE EDELSTAHLWERKE
AKTIENGESELLSCHAFT (VEW)
Elisabethstrasse 12
A-1010 Wien(AT)**

(72) Erfinder: **Miksitz, Franz
Agnesgasse 18
A-2630 Ternitz(AT)**

(74) Vertreter: **Widtmann, Georg, Dr.
Vereinigte Edelstahlwerke Aktiengesellschaft (VEW)
Elisabethstrasse 12
A-1010 Wien(AT)**

(54) **Verfahren zur Sterilisation.**

(57) Die Erfindung betrifft ein Verfahren zur Sterilisation von in Behältern abgefüllten Flüssigkeiten mit einem Wärmeträgermedium, z.B. Wasserdampf, wobei eine Temperatur über einen trägen Temperaturmeßfühler gemessen wird und der Druck im Sterilisator über einen Meßfühler durch Zufuhr eines Druckmediums, z.B. Druckluft, sowie die Zufuhr des Wärmeträgermediums über einen empfindlichen Temperaturmeßfühler im Sterilisator gesteuert wird, wobei die Temperaturmeßwerte des trägen Meßfühlers und des empfindlichen Temperaturmeßfühlers laufend verglichen werden und die Druckmittelzu- bzw. -ableitung über den zeitlichen Verlauf der beiden Temperaturen zumindest während der Aufheiz- und/oder Sterilisationszeit gesteuert wird.

Fig. 1

EP 0 134 777 A2

<u>Verfahren zur Sterilisation</u>

Die Erfindung hat ein Verfahren zur Sterilisation von in Behältern abgefüllten Flüssigkeiten mit einem Wärmeträgermedium zum Gegenstand.

Zur Hitzesterilisation ist die Einhaltung einer bestimmten Sterilisationsdauer von besonders hoher Bedeutung. Diese Sterilisationsdauer bestimmt sich jedoch nicht primär durch das Einwirken, z.B. des Wärmeträgermediums, auf das zu sterilisierende Gut sondern maßgeblich ist die Temperatur des Gutes wobei auch hier noch ein Temperaturgradient im zu sterilisierenden Gut zu berücksichtigen ist, da dasselbe zumindest in der Aufheizphase im Inneren eine geringere Temperatur aufweisen wird als an der Oberfläche, die direkt mit dem Wärmeträgermedium beaufschlagt wird.

Werden Flüssigkeiten oder Lösungen in verschlossenen Behältern sterilisiert so muß zusätzlich zur Wärmezufuhr im Sterilisator ein Überdruck aufgebaut werden. In der Regel sind die zu sterilisierenden Lösungen wäßrige Lösungen, deren Dampfdruck bei Temperaturen über 100 $^{o}$C größer als Atmosphärendruck ist, sodaß es zur Zerstörung der Behälter durch Explosion derselben kommen kann. Um dies zu vermeiden, wird während der Sterilisation im Sterilisator ein Gegendruck, z.B. mit Preßluft od.dgl., aufgebaut.

Es ist bereits ein Sterilisationsverfahren bekanntgeworden, bei dem in einem Sterilisator ein Temperaturmeßfühler in einer Referenzflasche angeordnet ist. Mit Erreichen der erwünschten Sterilisationstemperatur in der Referenzflasche wird der Sterilisationsbeginn festgelegt. Diese Referenzflasche weist einen im wesentlichen analogen Aufbau wie das zu sterilisierende Gut auf, d.h. es besteht aus einem Behälter, in welchem Flüssigkeit angeordnet ist, in die ein Meßfühler, z.B. Thermoelement od.dgl., ragt. Ein derartiger Meßfühler erlaubt annähernd die Verhältnisse im zu sterilisierenden Gut zu simulieren, erfordert jedoch, daß bei Wechsel des zu sterilisierenden Gutes auch die Referenzflasche gewechselt werden muß, was einen besonders großen Aufwand bedeutet oder - und in dieser Weise wurde bislang verfahren - es müssen die Sterilisationszeiten mit entsprechend großen Sicherheiten versehen werden, wodurch die Durchsatzleistungen der Sterilisatoren verringert und der Energieaufwand pro Sterilisation höher als erforderlich ist. Gleichzeitig wird bei diesem Verfahren ein Druckmeßfühler in einer geschlossenen Referenzflasche angeordnet, womit eine entsprechende Drucksteuerung erfolgen kann. In diesem Druckmeßfühler wird in etwa der Druck, der im zu sterilisierenden Gut herrscht, simuliert.

Die Zufuhr des Dampfes wird über einen Temperaturmeßfühler geregelt, welcher die augenblickliche Temperatur im Sterilisator mißt. Je nachdem, ob diese Temperatur über oder unter dem erwünschten Wert liegt, wird der freie Ventilquerschnitt für die Dampfzufuhr verkleinert bzw. vergrößert.

Diesem oben angeführten Verfahren haftet jedoch der Mangel an, daß im Sterilisator über Referenzflaschen ähnliche Verhältnisse simuliert werden müssen, wie sie im zu sterilisierenden Gut herrschen. Diese Referenzflaschen müssen jedoch im Sterilisator so untergebracht werden, daß eine leichte Beschickung und Entladung des Sterilisators ohne Zerstörung bzw. Gefährdung der Referenzflaschen stattfinden kann. Damit müssen die Referenzflaschen im Randbereich angeordnet werden, welcher einen anderen Temperaturverlauf aufweist als dies bei frei im Raum befindlichem zu sterilisierenden Gut gegeben ist.

Die Aufgabe der vorliegenden Erfindung besteht nun darin, die oben angeführten Nachteile zu vermeiden und ein Verfahren zur Sterilisation zu schaffen, bei dem die Steuerung lediglich über zwei laufend zu bestimmende Temperaturen und die Zeit erfolgt, wobei eine Sterilisation von verschiedenen Behältern in aufeinanderfolgender Reihenfolge - ohne mechanische Veränderung im Sterilisator - vorgenommen werden kann, und ohne daß unnötige Sicherheitszeiten in die Sterilisationszeit miteinbezogen werden müssen.

Das erfindungsgemäße Verfahren zur Sterilisation von in Behältern abgefüllten Flüssigkeiten mit einem Wäremträgermedium, z.B. Wasserdampf, wobei eine Temperatur über einen trägen Temperaturmeßfühler gemessen wird und der Druck im Sterilisator über einen Meßfühler durch Zufuhr eines Druckmediums, z.B. Druckluft, sowie die Zufuhr des Wärmeträgermediums über einen empfindlichen Temperaturmeßfühler im Sterilisator gesteuert wird, besteht im wesentlichen darin, daß die Temperaturmeßwerte des trägen Meßfühlers und des empfindlichen Temperaturmeßfühlers laufend verglichen werden und die Druckmittelzu- bzw. -ableitung über den zeitlichen Verlauf der beiden Temperaturen zumindest während der Aufheiz- und/oder Sterilisationszeit gesteuert wird.

Durch den zeitlichen Vergleich der gemessenen Temperaturen des trägen Meßfühlers und des empfindlichen Meßfühlers kann die augenblickliche Temperatur, die im zu sterilisierenden Gut herrscht, errechnet werden, wobei eine Apparatkonstante vorher zu bestimmen ist. Aus der so ermittelten Temperatur kann direkter Rückschluß auf den im Behälter herrschenden Druck gezogen werden, der sodann zur Steuerung der Druckmediumzufuhr dient. Mit einem derartigen Verfahren ist es daher möglich, lediglich über zwei Temperaturwerte und deren zeitlichen Verlauf einen gesamten Sterilisationsvorgang zu steuern.

Eine besonders empfindliche und daher gut reproduzierbare Sterilisation tritt dann ein, wenn zumindest während der Aufheiz- und/oder Sterilisationszeit die Zufuhr des Wärmeträgermediums über den empfindlichen Temperaturmeßfühler gesteuert wird.

Wird die Zufuhr des Wärmeträgermediums über den zeitlichen Verlauf der Temperaturmeßwerte des trägen und des empfindlichen Temperaturmeßfühlers und der vorgegebenen Sterilisationszeit unterbrochen, so können auch Störungen, die sich während des Aufheizvorganges ergeben, z.B. zu geringe Wärmeträgermediumzufuhr, wobei allerdings die Temperatur bereits über 100 °C beträgt, bei der zu ermittelnden Sterilisationszeit berücksichtigt werden, sodaß unnötige Überzeiten auch hier vermeidbar sind.

Wird die Druckmittelzufuhr zusätzlich über den zeitlichen Verlauf der Temperaturmeßwerte des trägen und des empfindlichen Temperaturmeßfühlers und der vorgegebenen Sterilisationszeit gesteuert, so kann einerseits eine unerwünschte Zerstörung der die zu sterilisierenden Flüssigkeiten aufweisenden Behälter vermieden werden und es ist gleichzeitig möglich, z.B. vor Ende der Sterilisationszeit, einen erwünschten zusätzlichen Überdruck aufzubauen.

Gemäß einem weiteren Merkmal der vorliegenden Erfindung wird die Temperatur im Sterilisator mit einem trägen Meßfühler gemessen, dessen kalorischer Wert kleiner ist als jener des zu sterilisierenden Gutes. Durch diese Vorgangsweise wird eine besonders empfindliche Steuerung der Sterilisation ermöglicht.

Im folgenden wird die Erfindung an Hand der Zeichnungen näher erläutert.

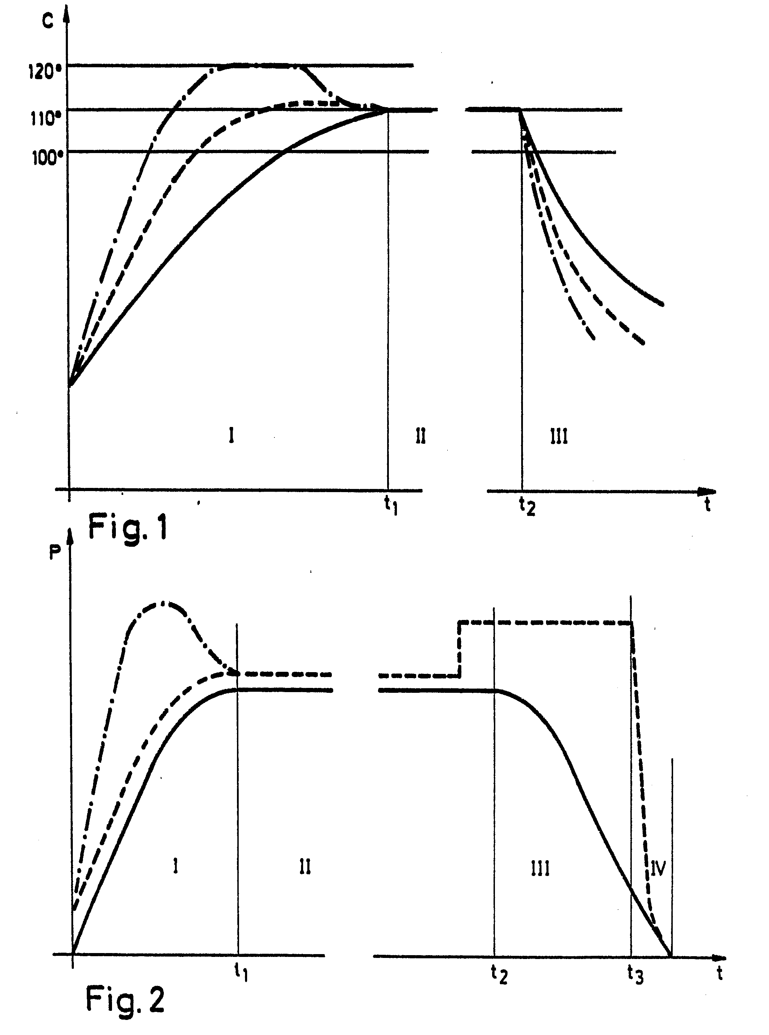

Die Figuren 1 und 2 zeigen den Temperatur- bzw. Druckverlauf während des Sterilisationsvorganges.

Die Temperatur im Sterilisator wird direkt von einem Thermoelement gemessen, das einen besonders kleinen kalorischen Wert, z.B. 2 cal, aufweist. Der träge Meßfühler wird in einem Meßrohr angeordnet, in dem 20 cm$^3$ eines Öles vorgelegt sind, in das ein weiteres Thermoelement eintaucht.

Der kalorische Wert dieses Meßfühlers beträgt ca. 20 cal. Auf Grund der verschiedenen kalorischen Werte, tritt zumindest während der Aufheiz- und Abkühlphase eine Temperaturdifferenz zwischen den beiden Werten auf, welche einen Rückschluß auf die im zu sterilisierenden Medium herrschende Temperatur erlaubt. Der Erwärmungsvorgang verläuft nach der Formel

$$\vartheta = \vartheta_{max} \cdot (1 - e^{\frac{-t}{\tau}})$$

der Abkühlvorgang hingegen nach der Formel

$$\vartheta = \vartheta_{max} \cdot (e^{\frac{-t}{\tau}})$$

$\tau$ ist eine Konstante, die empirisch ermittelt werden muß, in welche die Beschaffenheit des Sterilisators und des zu sterilisierenden Gutes samt Behälter eingeht, worin $\vartheta$ die Temperatur im zu sterilisierenden Gut $\vartheta_{max}$ die Temperatur im Sterilisator, also den Meßwert des empfindlichen Fühlers, darstellt, und t die Zeit bedeutet.

Durch den laufenden Vergleich der Temperaturwerte des trägen und des empfindlichen Fühlers können jeweils Störungen ermittelt werden, da beispielsweise die Temperatur des trägen Meßfühlers während des Aufheizvorganges und auch während der Sterilisation nie höher sein darf als jene des empfindlichen Fühlers. Die Sterilisationszeit beginnt ab Erreichen der erwünschten Temperatur im Gut zu laufen oder es kann gegebenenfalls bereits die Zeit mitberücksichtigt werden, ab welcher eine bestimmte Temperatur, z.B. 100 $^{\circ}$C, erreicht ist, da bei dieser Temperatur ebenfalls bereits eine Sterilisation bedingt wird. Die elektronische Steuerung des Vorganges erfolgt nun derart, daß einerseits laufend die beiden Temperaturen verglichen werden, um Störungen aufzuzeigen, andererseits die Temperatur im Meßgut berechnet und an Hand dieser Temperatur die Dauer der Sterilisation gesteuert wird. Weiters wird auf Grund der errechneten Temperatur im zu sterilisierenden Gut der Druck berrechnet, welcher sodann über ein entsprechendes

Druckregelventil, das zum Einlaß von Preßluft dient, steuerbar ist.

Bei dem in Fig. 1 dargestellten Diagramm handelt es sich um den Temperaturverlauf, und zwar wie er in den verschiedenen Phasen - I = Aufheizphase, II = Sterilisationsphase, III = Abkühlphase - gegeben ist. Hiebei ist strichpunktiert die Temperatur angegeben, wie sie im Sterilisator herrscht und durch die Meßwerte des empfindlichen Temperaturmeßfühlers wiedergegeben werden; strichliert die Temperatur wie sie mit dem trägen Temperaturmeßfühler gemessen wird; hingegen als durchgehende Linie die Temperatur des zu sterilisierenden Gutes. In der Phase I wird mit einer Übertemperatur gearbeitet, da ein möglichst schnelles Aufheizen erwünscht ist, die durch die Überhöhung der strichpunktierten Linie klar erkennbar ist. Die strichlierte Linie, die den Wert des trägen Meßfühlers wiedergibt, der einen geringeren kalorischen Wert aufweist als das zu sterilisierende Gut, kommt unterhalb der strichpunktierten Linie zu liegen, da ein Aufheizen des Fühlers erforderlich ist. Im zu sterilisierenden Gut, das einen noch größeren kalorischen Wert aufweist, liegt die Temperatur noch darunter. Mit Erreichen der erwünschten Temperatur, z.B. 110 $^{\circ}$C, beginnt die Sterilisationszeit zu laufen, es sei denn, es wird bereits die Zeitspanne mitberücksichtigt, wenn das Gut 100 $^{\circ}$C erreicht hat, da bei dieser Temperatur ebenfalls ein Abtötungseffekt vorhanden ist. Alle diese Daten können einem Prozeßrechner eingegeben werden, welcher sodann die jeweils erforderlichen Steuerungen vornimmt. Ist der Zeitpunkt $t_1$ erreicht, so muß lediglich so viel Wärme zugeführt werden wie durch Abstrahlung verlorengeht, d.h. die Temperaturdifferenz zwischen dem zu sterilisierenden Gut, dem trägen Temperaturmeßfühler und dem empfindlichen Temperaturmeßfühler wird nahezu null sein. Ist der Zeitpunkt $t_2$, d.h. der Endpunkt der Sterilisation erreicht, so wird die Zufuhr des Dampfes unterbrochen, wodurch die Temperatur im Sterilisator, wie an Hand der strichpunktierten Linie zu erkennen, rapide absinkt. Die Temperatur des trägen Meßfühlers (strichliert dargestellt) sinkt rascher als jene des zu sterilisierenden Gutes, da der kalorische Wert desselben geringer ist.

In Fig. 2 ist der zeitliche Druckverlauf dargestellt, wobei in der Phase I strichpunktiert der tatsächliche Kammerdruck angegeben und die Erhöhung auf die Expansion des im Sterilisator befindlichen Gases, z.B. Luft, zurückzuführen ist. Der im zu sterilisierenden Medium herrschende Druck ist als durchgehende Linie, wohingegen der mindesterforderliche Druckverlauf strichliert dargestellt ist. Wie ersichtlich, werden die zu sterilisierenden Behälter von außen mit einem Stützdruck beaufschlagt, der größer ist als der im Gut herrschende Druck. Kurz vor Sterilisationsende (Zeitpunkt $t_2$) wird eine zusätzliche Druckerhöhung aufgebracht, worauf die Zufuhr des Dampfes unterbrochen wird, und die Phase III - die Abkühlphase - eintritt, während welcher der überhöhte Druck aufrechterhalten wird. Zum Zeitpunkt $t_3$ beginnt die sogenannte Abströmphase IV, welche in der Belüftung des Sterilisators endigt.

Patentansprüche:
=================================

1. Verfahren zur Sterilisation von in Behältern abgefüllten Flüssigkeiten mit einem Wärmeträgermedium, z.B. Wasserdampf, wobei eine Temperatur über einen trägen Temperaturmeßfühler gemessen wird und der Druck im Sterilisator über einen Meßfühler durch Zufuhr eines Druckmediums, z.B. Druckluft, sowie die Zufuhr des Wärmeträgermediums über einen empfindlichen Temperaturmeßfühler im Sterilisator gesteuert wird, dadurch gekennzeichnet, daß die Temperaturmeßwerte des trägen Meßfühlers und des empfindlichen Temperaturmeßfühlers laufend verglichen werden und die Druckmittelzu- bzw. -ableitung über den zeitlichen Verlauf der beiden Temperaturen zumindest während der Aufheiz- und/oder Sterilisationszeit gesteuert wird.

2. Verfahren nach Anspruch 1, dadurch gekennzeichnet, daß zumindest während der Aufheiz- und/oder Sterilisationszeit die Zufuhr des Wärmeträgermediums über den empfindlichen Temperaturmeßfühler gesteuert wird.

3. Verfahren nach Anspruch 1 oder 2, dadurch gekennzeichnet, daß die Zufuhr des Wärmeträgermediums über den zeitlichen Verlauf der Temperaturmeßwerte des trägen und des empfindlichen Temperaturmeßfühlers und der vorgegebenen Sterilisationszeit unterbrochen wird.

4. Verfahren nach Anspruch 1, 2 oder 3, dadurch gekennzeichnet, daß die Druckmittelzufuhr zusätzlich über den zeitlichen Verlauf der Temperaturmeßwerte des trägen und des empfindlichen Temperaturmeßfühlers und der vorgegebenen Sterilisationszeit gesteuert wird.

5. Verfahren nach einem der Ansprüche 1 bis 4, dadurch gekennzeichnet, daß die Temperatur im Sterilisator mit einem trägen Meßfühler gemessen wird, dessen kalorischer Wert kleiner ist als jener des zu sterilisierenden Gutes.

1/1

0134777

Fig. 1

Fig. 2